# EUROPEAN PATENT APPLICATION

(11) **EP 4 353 228 A1**
(43) Date of publication of application: **17.04.2024**
(21) Application number: 22819578.0
(22) Date of filing: 08.06.2022
(51) Int. Cl.: A61K 31/4164, A61K 31/49, A61K 31/135, A61K 31/4525, A61P 33/14, A61P 27/02

(54) **USE OF COMPOUNDS IN INHIBITING OR KILLING MITES AND TREATING DRY-EYE SYNDROME**

(30) Priority: 08.06.2021 CN 202110649319; 08.06.2021 CN 202110649315; 11.06.2021 CN 202110653838; 11.06.2021 CN 202110653692; 11.06.2021 CN 202110659857
(71) Applicant: Precvision Biotechnologies Ltd., Guangzhou, Guangdong 510000 (CN)
(72) Inventor: WEI, Lai, Guangzhou, Guangdong 510000 (CN)
(74) Representative: J A Kemp LLP
(86) International application number: PCT/CN2022/097672
(87) International publication number: WO 2022/257981

(57) **Abstract**

Provided is a method for killing mites and treating xerophthalmia by compounds and salts, stereoisomers and solvates thereof.

## Description

### FIELD

The present invention relates to the field of medicine, and in particular to a use of a compound in inhibiting or killing mites and treating xerophthalmia.

### BACKGROUND

Mite-induced diseases (for example, eye diseases and skin diseases) have not been paid enough attention in clinical practice, and are often misdiagnosed as bacterial diseases; however, conventional antibacterial treatment often has no obvious effect.

### SUMMARY

To overcome the deficiencies of the prior art, the present invention provides a use of a compound, a salt, a stereoisomer, and a solvate thereof in the preparation of products for inhibiting and/or killing mites.

In one embodiment of the present invention, the compound is of the following formula: 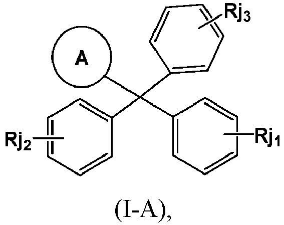
wherein Rj₁, Rj₂, and Rj₃ are one or more arbitrary and independent substituents on the corresponding phenyl group; Rj₁, Rj₂, and Rj₃ each is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂; and
ring A is a heterocyclic group.

Specifically, the ring A may be a five-membered or six-membered heteroaryl including 1, 2, or 3 heteroatoms selected from N, O, or S atoms.

In one embodiment of the present invention, the ring A is 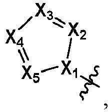 wherein, X₁, X₂, X₃, X₄, and X₅ are independently selected from CRj₄ or N;
Rj₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

Specifically, the ring A may be selected from 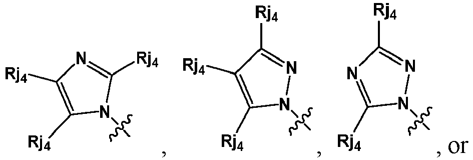 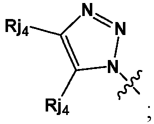 each Rj₄ is independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

In one example of the present invention, Rj₄ is H.

In another embodiment of the present invention, the ring A is 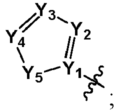 Y₁, Y₂, Y₃, and Y₄ are independently selected from CRj₅ or N; Y₅ is selected from O, S, NRj₅, or CRj₅Rj₆;
Rj₅ and Rj₆ are independently of each other selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

Specifically, the ring A may be selected from 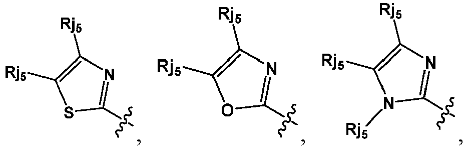 or

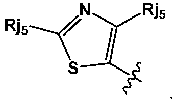

In one example of the present invention, Rj₅ is H.

Specifically, Rj₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rj₁ is Cl.

Specifically, Rj₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rj₂ is H.

Specifically, Rj₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rj₃ is H.

In one embodiment of the present invention, the above compound has the following structure: 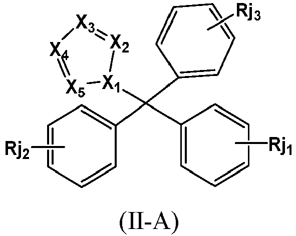 wherein, Rj₁, Rj₂, Rj₃, X₁, X₂, X₃, X₄, and X₅ have the above corresponding definitions of the present invention.

Specifically, the above compound has the following structure: 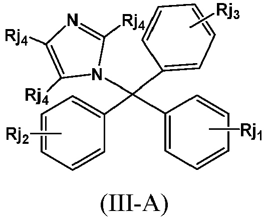 wherein, Rj₁, Rj₂, Rj₃, and Rj₄ have the above corresponding definitions of the present invention.

More specifically, the above compound has the following structure: 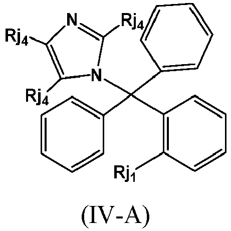 wherein, Rj₁ and Rj₄ have the above corresponding definitions of the present invention. In one example of the present invention, the above compound has the following structure:

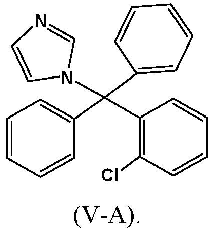

In another embodiment of the present invention, the compound has the following structure: 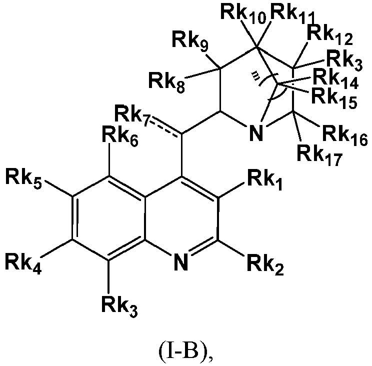 wherein,
Rk₁, Rk₂, Rk₃, Rk₄, Rk₅, and Rk₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; or, Rk₁ and Rk₂ form an aryl or heterocyclic group together with carbon atoms to which they are attached; two of Rk₃, Rk₄, Rk₅, and Rk₆ form an aryl or heterocyclic group together with carbon atoms to which they are attached;
-̅ -̅ -̅ -̅ -̅ represents a single bond or a double bond;
Rk₇ is selected from H, O, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂;
Rk₈, Rk₉, Rk₁₀, Rk₁₁, Rk₁₂, Rk₁₃, Rk₁₄, Rk₁₅, Rk₁₆, and Rk₁₇ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂; and
m is selected from an integer from 1 to 5.

Specifically, Rk₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁ is H.

Specifically, Rk₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₂ is H.

Specifically, Rk₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₃ is H.

Specifically, Rk₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₄ is H.

Specifically, Rk₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; specifically, R5 is OH or O(C₁₋₈ alkyl), such as OCH₃, O(C₂ alkyl), O(C₃ alkyl), O(C₄ alkyl) O(C₅ alkyl), O(C₆ alkyl), O(C₇ alkyl), O(C₈ alkyl); in one example of the present invention, Rk₅ is OCH₃.

In one example of the present invention, -̅ -̅ -̅ -̅ -̅ is a single bond.

Specifically, Rk₇ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; more specifically, Rk₇ is OH or O(C₁₋₈ alkyl), such as OCH₃, O(C₂ alkyl), O(C₃ alkyl), O(C₄ alkyl) O(C₅ alkyl), O(C₆ alkyl), O(C₇ alkyl)CO(C₈ alkyl); in one example of the present invention, Rk₇ is OH.

Specifically, m is selected from 1, 2, 3, 4, or 5; in one example of the present invention, m is 2.

Specifically, Rk₈ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₈ is H.

Specifically, Rk₉ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₉ is H.

Specifically, Rk₁₀ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₀ is H.

Specifically, Rk₁₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₁ is H.

Specifically, Rk₁₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₂ is H.

Specifically, Rk₁₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, or C₂₋₈ alkenyl; more specifically, Rk₁₃ is selected from C₂₋₈ alkenyl; in one example of the present invention, Rk₁₃ is ethenyl.

Specifically, Rk₁₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₄ is H.

Specifically, Rk₁₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₅ is H.

Specifically, Rk₁₆ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₆ is H.

Specifically, Rk₁₇ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rk₁₇ is H.

In one embodiment of the present invention, the above compound has the following structure: 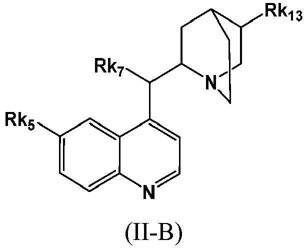 wherein, Rk₅, Rk₇, and Rk₁₃ have the above corresponding definitions of the present invention.

In one example of the present invention, the above compound has the following structure:

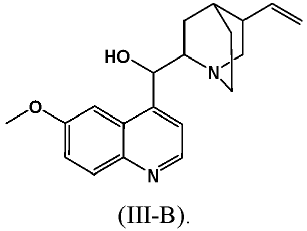

In one example of the present invention, the above compound has the following structure:

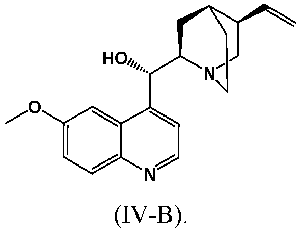

In one example of the present invention, the salt of the above compound is a sulfate salt thereof.

In another embodiment of the present invention, the compound has the following structure: 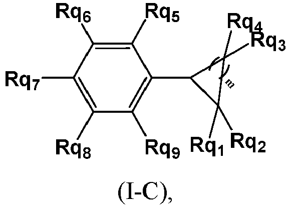 wherein,
Rq₁, Rq₂, Rq₃, and Rq₄ are each independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂;
Rq₅, Rq₆, Rq₇, Rq₈, and Rq₉ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), NHC(=O)(C₆₋₁₈ aryl), NHC(=0)(C1-10 aralkyl), NHC(=0)(C1-10 arylalkoxy), N(C₁₋₈ alkyl)C(=O)(C₁₋₈ alkyl), NHC(=O)(C₁₋₈ alkyl)NHC(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, two of Rq₁, Rq₂, Rq₃, Rq₄, Rq₅, Rq₆, Rq₇, Rq₈, and Rq₉ (for example, Rq₅ and Rq₆, Rq₆ and Rq₇, Rq₇ and Rq₈, Rq₈ and Rq₉, Rq₉ and Rq₁, Rq₉ and Rq₂, Rq₅ and Rq₄, Rq₅ and Rq₃, Rq₃ and Rq₂, Rq₃ and Rq₁, Rq₄ and Rq₂, and Rq₄ and Rq₁) form an aryl, cycloalkyl, or heterocyclic group together with carbon atoms to which they are attached; and
m is selected from an integer from 1 to 5.

Specifically, Rq₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; more specifically, Rq₁ is selected from NH₂, NH(C₁₋₈ alkyl), N(C₁₋₈ alkyl)₂; in one example of the present invention, Rq₁ is NH₂.

Specifically, Rq₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₂ is H.

Specifically, m is selected from 1, 2, 3, 4, or 5; in one example of the present invention, m is 1.

Specifically, each Rq₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₃ is H.

Specifically, each Rq₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₄ is H.

Specifically, Rq₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₅ is H.

Specifically, Rq₆ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₆ is H.

Specifically, Rq₇ is selected from H, F, Cl, Br, I, C₁₋₈ alkyl, O(C₁₋₈ alkyl), NH(C₁₋₈ alkyl), N(C₁₋₈ alkyl)₂, NHC(=O)(C₁₋₈ alkyl), NHC(=O)(C₆₋₁₈ aryl), NHC(=O)(C1-10 aralkyl), NHC(=O)(C1-10 arylalkoxy), OH, NH₂, SH, CO₂H, CN, CF₃, CHF₂, CH₂F, or NO₂; in one example of the present invention, Rq₇ is H.

Specifically, Rq₈ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₈ is H.

Specifically, Rq₉ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rq₉ is H.

Specifically, the above compound has the following structure: 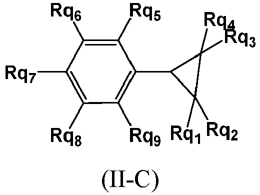 wherein, Rq₁, Rq₂, Rq₃, Rq₄, Rq₅, Rq₆, Rq₇, Rq₈, and Rq₉ have the above corresponding definitions of the present invention.

In one embodiment of the present invention, the above compound has the following structure: 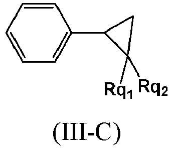 wherein, Rq₁ and Rq₂ have the above corresponding definitions of the present invention.

In one example of the present invention, the above compound has the following structure:

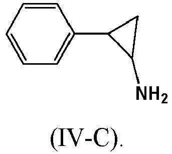

In one example of the present invention, the above compound has the following structure:

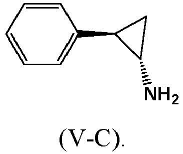

In one example of the present invention, the salt of the above compound is a hydrochloride salt thereof.

In another embodiment of the present invention, the compound has the following structure: 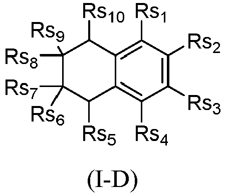 wherein, Rs₁, Rs₂, Rs₃, Rs₄, Rs₅, Rs₆, Rs₇, Rs₈, Rs₉, and Rs₁₀ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂.

Further, the compound has the following structure: 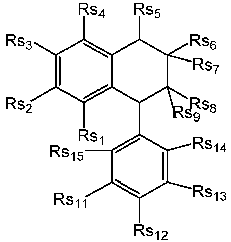 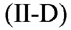 wherein, Rs₁₁, Rs₁₂, Rs₁₃, Rs₁₄, and Rs₁₅ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C_{I-8} alkyl), or NHSO₂N(C₁₋₈ alkyl)₂.

Specifically, Rs₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₁ is H.

Specifically, Rs₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₂ is H.

Specifically, Rs₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₃ is H.

Specifically, Rs₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₄ is H.

Specifically, Rss is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
further, Rss is selected from NH(C₁₋₈ alkyl); in one example of the present invention, Rss is NHCH₃.

Specifically, Rs₆ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₆ is H.

Specifically, Rs₇ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, R_{S7} is H.

Specifically, Rs₈ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₈ is H.

Specifically, Rs₉ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₉ is H.

Specifically, Rs₁₀ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, or C₂₋₈ alkynyl;
further, Rs₁₀ is selected from aryl or heteroaryl.

Specifically, Rs₁₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
Further, Rs₁₁ is H, F, Cl, Br, or I; in one example of the present invention, Rs₁₁ is Cl.

Specifically, Rs₁₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl.

Further, Rs₁₂ is H, F, Cl, Br, or I; in one example of the present invention, Rs₁₂ is Cl.

Specifically, Rs₁₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₁₃ is H.

Specifically, Rs₁₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₁₄ is H.

Specifically, Rs₁₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl; in one example of the present invention, Rs₁₅ is H.

In one embodiment of the present invention, the above compound has the following structure: 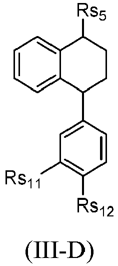 wherein, Rss, Rs₁₁, and Rs₁₂ have the above corresponding definitions of the present invention.

In one example of the present invention, the above compound has the following structure:

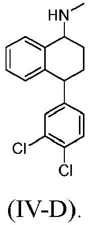

In one example of the present invention, the above compound has the following structure:

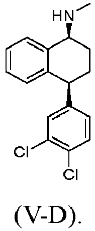

In one example of the present invention, the salt of the above compound is a hydrochloride salt thereof.

In another embodiment of the present invention, the compound has the following structure: 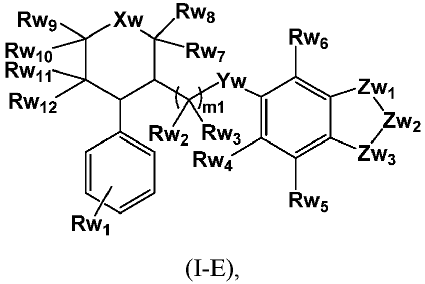 wherein,
Rw₁ is one or more arbitrary and independent substituents on the phenyl, each Rw₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rw₂, Rw₃, Rw₄, Rw₅, Rw₆, Rw₇, Rw₈, Rw₉, Rw₁₀, Rw₁₁, and Rw₁₂ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, Rw₃ and Rw₄, or Rw₄ and Rw₅ form an aryl, cycloalkyl or heterocyclic group together with carbon atoms to which they are attached;
Xw, Yw, Zw₁, Zw₂, and Zw₃ are independently selected from CRw₁₃Rw₁₄, O, S, or NRw₁₃;
m1 is selected from an integer from 1 to 5; and

Rw₁₃ and Rw₁₄ are independently of each other selected from H, halogen, C₁₋₈ alkyl, C₃₋₁₁ cycloalkyl, aryl, or heteroaryl.

Specifically, Rw₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₁ is F.

Specifically, Rw₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₂ is H.

Specifically, Rw₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₃ is H.

Specifically, Rw₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₄ is H.

Specifically, Rw₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₅ is H.

Specifically, Rw₆ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₆ is H.

Specifically, Rw₇ and Rw₈ are independently selected H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₇ is H; in one example of the present invention, Rw₈ is H.

Specifically, Rw₉ and Rw₁₀ are independently selected H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₉ is H; in one example of the present invention, Rw₁₀ is H.

Specifically, Rw₁₁ and Rw₁₂ are independently selected H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rw₁₁ is H; in one example of the present invention, Rw₁₂ is H.

Specifically, m1 is selected from 1, 2, 3, 4, or 5; in one example of the present invention, m1 is 1.

In one embodiment of the present invention, Xw is NRw₁₃; Rw₁₃ may be selected from H, C₁₋₈ alkyl, or C₃₋₁₁ cycloalkyl; in one example of the present invention, Xw is NH.

In one embodiment of the present invention, Yw is O.

In one embodiment of the present invention, Zw₁ is O.

In one embodiment of the present invention, Zw₂ is CRw₁₃Rw₁₄; wherein, Rw₁₃ and Rw₁₄ may be independently selected from H, halogen, C₁₋₈ alkyl, C₃₋₁₁ cycloalkyl, aryl, or heteroaryl; in one example of the present invention, Zw₂ is CH₂.

In one embodiment of the present invention, Zw₃ is O.

In one embodiment of the present invention, the above compound has the following structure: 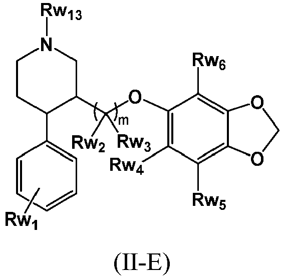 wherein, Rw₁, Rw₂, Rw₃, Rw₄, Rw₅, Rw₆, and Rw₁₃ have the above corresponding definitions of the present invention.

More specifically, the above compound has the following structure: 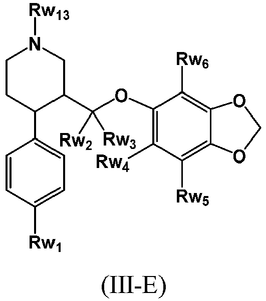 wherein, Rw₁, Rw₂, Rw₃, Rw₄, Rw₅, Rw₆, and Rw₁₃ have the above corresponding definitions of the present invention.

More specifically, the above compound has the following structure: 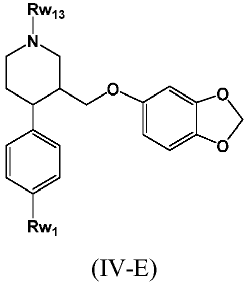 wherein, Rw₁ and Rw₁₃ have the above corresponding definitions of the present invention.

In one example of the present invention, the above compound has the following structure:

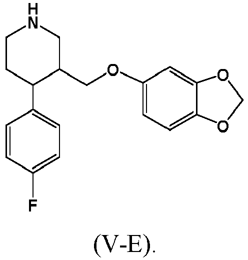

In one example of the present invention, the above compound has the following structure:

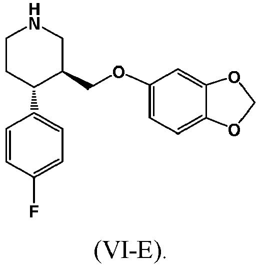

In one example of the present invention, the salt of the above compound is a hydrochloride salt thereof.

In another embodiment of the present invention, the compound has the following structure: 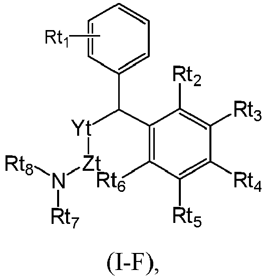 wherein,
Rt₁ is one or more arbitrary and independent substituents on the phenyl, each Rt₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₂, Rt₃, Rt₄, Rt₅, and Rt₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₇ and Rt₈ are independently selected from H, C₁₋₈ alkyl, or C₃₋₆ cycloalkyl;
Yt is selected from CRt₉Rt₁₀, O, S, or NRt₉;
Zt is C₁₋₈ alkylene; and
Rt₉ and Rt₁₀ are independently of each other selected from H, halogen, C₁₋₈ alkyl, C₃₋₁₁ cycloalkyl, aryl, or heteroaryl.

Specifically, each Rt₁ is independently selected H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rt₁ is H.

Specifically, Rt₂ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; Specifically, Rt₂ is selected from H, C₁₋₃ alkyl (for example, methyl, ethyl, n-propyl, or isopropyl), CF₃, CHF₂, or CH₂F; in one example of the present invention, Rt₂ is methyl.

Specifically, Rt₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rt₃ is H.

Specifically, Rt₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rt₄ is H.

Specifically, Rt₅ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rt₅ is H.

Specifically, Rt₆ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; in one example of the present invention, Rt₆ is H.

Specifically, Rt₇ and Rt₈ are independently selected from H and C₁₋₃ alkyl (for example, methyl, ethyl, or N-propyl); in one example of the present invention, Rt₇ is methyl; in one example of the present invention, Rt₈ is methyl.

Specifically, Yt is selected from CH₂, O, S, or NH; more specifically, Yt is O or S; in one example of the present invention, Yt is O.

Specifically, Zt is C₁₋₆ alkylene, in particular, C₁₋₃ alkylene, such as -CH₂-, -CH₂CH₂-, or - CH₂CH₂CH₂-; in one example of the present invention, Zt is -CH₂CH₂-.

In some examples of the present invention, the above compound has the following structure: 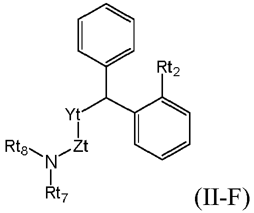 wherein, Rt₂, Rt₇, Rt₈, Yt, and Zt have the above corresponding definitions of the present invention.

More specifically, the above compound has the following structure: 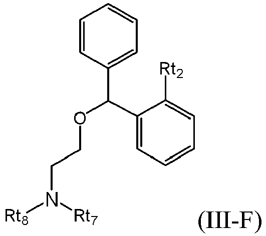 wherein, Rt₂, Rt₇, Rt₈, and Yt have the above corresponding definitions of the present invention.

In one example of the present invention, the above compound has the following structure:

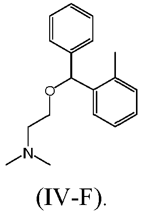

In one example of the present invention, the salt of the above compound is a citrate salt thereof.

Specifically, the above salts, stereoisomers, and solvates are pharmaceutically acceptable salts, stereoisomers, and solvates.

Specifically, the above compounds, or salts, stereoisomers, and solvates thereof may be used as the sole active ingredient or in combination with other same or different active ingredients for inhibiting or killing mites.

Specifically, the above acaricidal products according to the present invention can be used for therapeutic and/or prophylactic purposes as well as for non-therapeutic and/or prophylactic purposes.

Specifically, the above mites of the present invention may be one or more of Demodex, dust mites, mange mites, or the like. In an embodiment of the present invention, the above mites of the present invention are Demodex, such as Demodex folliculorum and Demodex brevis.

In an embodiment of the present invention, the above products for inhibiting and/or killing mites of the present invention are pharmaceutical compositions.

Specifically, the above pharmaceutical composition further includes a pharmaceutically acceptable excipient.

Specifically, the above pharmaceutical composition of the present invention can be used for the prevention and/or treatment of diseases caused by mite infection.

In one embodiment of the present invention, the above use is the use of the compound, or the salt, stereoisomer, and solvate thereof in the preparation of drugs for preventing and/or treating diseases caused by mite infections.

Specifically, the above diseases may be eye diseases, skin diseases, allergic diseases, or the like.

Specifically, the above eye diseases may be one or more of blepharitis, blepharial keratoconjunctivitis, meibomian gland dysfunction, eyelash shedding, abnormal eyelash arrangement, conjunctivitis, or blepharioconjunctivitis, pterygium, keratitis, basal cell carcinoma of eyelid, xerophthalmia, chalazion, or the like; it may have one or more symptoms selected from red itching of eyes, dry eyes, burning sensation of eyes, foreign body sensation, photophobia, increased eye secretion, drop of eyelashes, blurred vision, decreased vision, or the like.

Specifically, the above skin diseases may be one or more of seborrheic dermatitis, acne, rosacea, pityriasis folliculitis, perioral dermatitis, Demodex diseases, scabies, basal cell carcinoma, or the like.

Specifically, the allergic diseases may be one or more of allergic asthma, allergic rhinitis, allergic dermatitis, allergic conjunctivitis, or the like.

Specifically, the pharmaceutical compositions may be in any form suitable for administration, such as topical preparations, in particular ophthalmic preparations, topical dermatological preparations, or the like.

Specifically, the above ophthalmic preparations may be eye drops, eye ointments, ophthalmic gels, ophthalmic emulsions, ophthalmic suspensions, ophthalmic films, ophthalmic solutions, intraocular injections, or the like.

Specifically, the above topical dermatological preparations may be aerosols, powders, lotions, tinctures, liniments, films, ointments, gels, pastes, emulsions, or the like.

Specifically, various dosage forms of the above pharmaceutical compositions of the present invention can be prepared according to a conventional production method in the pharmaceutical field.

Specifically, the above pharmaceutical compositions of the present invention may contain 0.01 to 99.5% (specifically, for example, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%) by weight of the active ingredient.

Specifically, the above pharmaceutical compositions can be used in humans, as well as veterinary uses in non-human animals, for example, non-human mammals, for example, pet animals (for example, dogs, cats, rabbits, mice, or the like), or livestock animals (for example, horses, cows, sheep, pigs, or the like).

In another embodiment of the present invention, the above acaricidal product of the present invention is cosmetic.

Specifically, the above cosmetics also include cosmetically acceptable excipients.

Specifically, the above cosmetic of the present invention may be a cosmetic for the face, such as a facial wash, a soap, a toner, a toner, a skincare cream, a gel, a facial cream, a sunscreen cream, an essence, a pack, a gel, a foundation, a frosting cream, or the like.

Specifically, the cosmetic of the present invention may be a cosmetic for other parts than the face, such as a neck cream, a shampoo, a body wash, a perfumed soap, a hair conditioner, a body lotion, a frosting cream, or the like.

Specifically, the above cosmetic of the present invention may also be cosmetic for eyes and periocular, such as an eye cream, a mascara, an eyeliner powder, an eyeliner cream, an eyeliner pen, an eye shadow powder, an eye shadow cream, an eyebrow pen, an eyebrow powder, or the like.

Specifically, various forms of the above cosmetics of the present invention can be prepared according to a conventional production method in the field of cosmetics.

Specifically, the cosmetic of the present invention may contain the active ingredient in an amount of 0.01 to 99.5% by weight (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%).

In another embodiment of the present invention, the above acaricidal product of the present invention is an acaricide, which can be used for killing and inhibiting mites that may colonize an article (for example, pillowcase, pillow core, bed sheet, bedding, mattress, clothing, carpet, cushion, sofa, summer sleeping mat, plush toy, air conditioner, or the like) in a living environment.

Specifically, the acaricides described above may contain any suitable excipient that can achieve the desired properties.

Specifically, the above acaricide may be in the form of a spray, a lotion, a patch, a small package, or the like.

Specifically, various forms of the above acaricide of the present invention can be prepared according to a conventional production method in the daily care product field.

Specifically, the above acaricide of the present invention may contain 0.01 to 99.5% by weight (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%) of the active ingredient.

The present invention also provides a method of preventing and/or treating diseases caused by mite infection, including the step of administering to a subject in need thereof the above compound of the present invention or salts, stereoisomers and solvates thereof, and pharmaceutical compositions.

Specifically, in the above method, the diseases, the compounds, or salts, stereoisomers, and solvates thereof, and the pharmaceutical compositions have the above corresponding definitions of the present invention.

Specifically, the above subjects can be any animal receiving the prevention and/or treatment, especially mammals, such as humans, cats, dogs, rabbits, mice, horses, cattle, sheep, pigs, or the like. In an embodiment of the present invention, the subject is a human; in another embodiment of the present invention, the subject is a non-human animal.

Specifically, the amount of the above compound, or salts, stereoisomers and solvates thereof, or pharmaceutical composition administered may vary depending on such factors as the route of administration, age, weight of the subject, and types and severity of the disease to be treated.

The present invention also provides a cosmetic method including the step of administering to a subject in need thereof a compound shown in formula I-A, I-B, I-C, I-D, I-E, or I-F, or a salt, stereoisomer, and solvate thereof to ameliorate skin problems such as skin roughness, increased dandruff, or itching caused by mites in the subject.

Specifically, in the method, the subject is a human.

Specifically, the compound has the above corresponding definitions of the present invention.

The present invention also provides The use of the compound shown in formula I-A, I-B, I-C, I-D, I-E, or I-F, and a salt, stereoisomer, and solvate thereof in the preparation of drugs for preventing and/or treating xerophthalmia.

Specifically, the compound shown in formula I-A, I-B, I-C, I-D, I-E, or I-F and the salt, stereoisomer, and solvate thereof have the above corresponding definitions of the present invention.

Specifically, the compound shown in formula I-A, I-B, I-C, I-D, I-E, or I-F and the salt, stereoisomer, and solvate thereof may be used as the sole active ingredient or in combination with other same or different active ingredients for the prevention and/or treatment of xerophthalmia.

Specifically, xerophthalmia has one or more symptoms selected from eye itching, foreign body sensation, burning sensation, photophobia, blurred vision, fluctuated vision, dry eyes, easy fatigue of eyes, thick secretions, sensitivity to topical stimuli, redness and swelling of eyes, congestion, keratinization, broken corneal epithelium with filamentous adhesion, or the like.

In an embodiment of the present invention, the xerophthalmia described above is xerophthalmia caused by infection with mites or related to mite infection.

Specifically, the drugs described above can be used in humans, as well as veterinary uses in non-human animals, for example, non-human mammals, for example, pet animals (for example, dogs, cats, rabbits, mice, or the like), livestock animals (for example, horses, cows, sheep, pigs, or the like).

Specifically, the above drugs may also include suitable pharmaceutically acceptable excipients.

Specifically, the drug may be in any form suitable for administration, such as an topical preparation, particularly an ophthalmic preparation, or the like.

Specifically, the above ophthalmic preparations may be eye drops, eye ointments, ophthalmic gels, ophthalmic emulsions, ophthalmic suspensions, ophthalmic films, ophthalmic solutions, intraocular injections, or the like.

The present invention also provides a method of preventing and/or treating xerophthalmia, including the step of administering to a subject in need thereof the above compound shown in formula I-A, I-B, I-C, I-D, I-E, or I-F, and the salt, stereoisomer, and solvate thereof, and the drugs.

Specifically, in the above method, xerophthalmia, the above compound shown in formula I-A, I-B, I-C, I-D, I-E, or I-F, and the salt, stereoisomer, and solvate thereof, and the drugs have the above corresponding definitions of the present invention.

Specifically, the above subjects can be any animal receiving the prevention and/or treatment, especially mammals, such as humans, cats, dogs, rabbits, mice, horses, cattle, sheep, pigs, or the like. In an embodiment of the present invention, the subject is a human; in another embodiment of the present invention, the subject is a non-human animal.

Specifically, the amount of the above compound shown in formula I-A, I-B, I-C, I-D, I-E, or I-F, and the salt, stereoisomer, and solvate thereof, or the drugs to be administered may vary depending on factors such as the route of administration, age, weight of the subject, and types and severity of the disease to be treated.

Through extensive experiments, the inventors have found that the above compound shown in formula I-A, I-B, I-C, I-D, I-E, or I-F, and the salt, stereoisomer, and solvate thereof (especially the compound of formula V) can be used for mite inhibition and killing, can effectively shorten the survival time of demodex, and can be used for mite killing and inhibiting products (such as drugs, cosmetics, daily necessities, or the like). In addition, the inventors have discovered that the above compound shown in formula I-A, I-B, I-C, I-D, I-E, or I-F, and the salt, stereoisomer, and solvate thereof (especially the compound of formula V-A) are also useful for treating, effectively alleviating, and ameliorating symptoms of xerophthalmia.

### DETAILED DESCRIPTION

Unless defined otherwise, all scientific and technical terms used herein have the same meaning as commonly understood by the ordinarily skilled in the art to which the present invention belongs.

The term "alkyl" refers to a hydrocarbon chain radical which is straight or branched and containing no unsaturation bonds, and which is attached to the rest of the molecule by a single bond. C₁₋₈ alkyl includes C₁ alkyl (that is, methyl), C₂ alkyl (that is, ethyl), C₃ alkyl (for example, n-propyl, and isopropyl), C₄ alkyl (for example, n-butyl, isobutyl, and t-butyl), C₅ alkyl, C₆ alkyl, C₇ alkyl, and C₈ alkyl.

The term "alkenyl" refers to a straight or branched hydrocarbon chain radical containing at least two carbon atoms and at least one unsaturated bond, and which is attached to the rest of the molecule by a single bond. C₂₋₈ alkenyl includes C₂ alkenyl (that is, ethenyl), C₃ alkenyl (for example, 1-propenyl, 2-propenyl, and 1-methyl-ethenyl), C₄ alkenyl (for example, 1-butenyl, 2-butenyl, and 3-butenyl), C₅ alkenyl, C₆ alkenyl, C₇ alkenyl, and C₈ alkenyl.

The term "alkynyl" refers to a straight or branched hydrocarbon chain radical containing at least two carbon atoms and at least one carbon-carbon triple bond, and which is attached to the rest of the molecule by a single bond. C₂₋₈ alkynyl includes C₂ alkynyl (that is, ethynyl), C₃ alkynyl (for example, 1-propynyl and 2-propynyl), C₄ alkynyl (for example, 1-butynyl, 2-butynyl, and 3-butynyl), C₅ alkynyl, C₆ alkynyl, C₇ alkynyl, and C₈ alkynyl.

The term "cycloalkyl" refers to an alicyclic hydrocarbon such as cyclopropyl, cyclohexyl, adamantyl, or the like.

The term "aryl" refers to a monocyclic or polycyclic radical, including polycyclic radicals containing a monoaryl group and/or a fused aryl group, such as phenyl, naphthyl, biphenyl, indenyl, phenanthrenyl, anthracenyl, or the like.

The term "heterocyclic group" includes heteroaromatic and heteroalicyclic groups containing 1 to 3 monocyclic and/or fused rings and 3 to about 18 ring atoms, and the heterocyclic groups of the present invention containing 1, 2, or 3 heteroatoms selected from N, O, or S atoms. Heteroaryl includes, but are not limited to, coumarin, including 8-coumarin, quinolinyl, including 8-quinolinyl, isoquinolinyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothiophenyl, benzothiazolyl, benzoxazolyl, quinazolinyl, quinoxalinyl, naphthyridinyl, furopyridinyl and the like; heteroalicyclic groups include, for example, pyrrolidinyl, tetrahydrofuranyl, dihydrofuranyl, tetrahydrothiophenyl, tetrahydrothiopyranyl, piperidinyl, morpholinyl, thiomorpholinyl, oxathianyl, piperazinyl, azetidinyl, oxetanyl, thietanyl, homopiperidinyl, oxiranyl, thietanyl, azepinyl, oxazepinyl, diazepinyl, triazepinyl, 1,2,3,6-tetrahydropyridyl, 2-pyrrolidinyl, 3-pyrrolidinyl, dihydroindole, 2H-pyranyl, 4H-pyranyl, dioxacyclohexyl, 1,3-dioxopentyl, pyrazolyl, dithiyl, dithiopentyl, dihydropyranyl, dihydrothiophene, pyrazolyl, imidazolyl, imidazolidinyl, 3-azabicyclo[3.1.0]hexyl, 3-azabicyclo[4.1.0]heptyl, 3H-indole, quinazinyl, or the like.

Such groups may be substituted at one or more available positions with one or more suitable groups.

The term "halogen" includes fluorine, chlorine, bromine, and iodine.

The term "salt" shall be understood to mean any form of a compound used in accordance with the present invention, where the compound is in ionic form or is charged and coupled to an ion (cation or anion) with an opposite charge or in solution. Also included in the definition are quaternary ammonium salts and complexes of the molecule with other molecules and ions, particularly complexes formed by ionic interactions. The definition includes physiologically acceptable salts; the term is to be understood as equivalent to "pharmacologically acceptable salts" or "pharmaceutically acceptable salts".

Specifically, the pharmaceutically acceptable salts include acid addition salts and base addition salts.

Acid addition salts include, but are not limited to, salts derived from inorganic acids such as hydrochloric acid, nitric acid, phosphoric acid, sulfuric acid, hydrobromic acid, hydroiodic acid, and phosphonic acids, and salts derived from organic acids such as aliphatic mono-and dicarboxylic acids, phenyl-substituted alkanoic acids, hydroxyalkanoic acids, alkanedioic acids, aromatic acids, and aliphatic and aromatic sulfonic acids. Thus, these salts include, but are not limited to, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromide, iodate, formate, acetate, propionate, octanoate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenyl acetates, citrates, lactates, maleates, tartrates and methanesulfonates, or also salts of amino acids such as arginate, glutamate, glycinate, gluconate, galacturonate, or the like. Acid addition salts can be prepared by contacting the free base form with enough amount of the desired acid to produce the salt in the conventional manner. The free base form may be regenerated by contacting the salt form with a base and isolating the free base in the conventional manner.

Base addition salts are formed with metals or amines, such as hydroxides of alkali and alkaline earth metals, or with organic amines. Examples of metals used as cations include, but are not limited to, sodium, potassium, magnesium, and calcium. Examples of suitable amines include, but are not limited to, N,N'-dibenzylethylene diamine, chloroprocaine, choline, diethanolamine, ethylenediamine (ethane-1,2-diamine), N-methylglucamine, and procaine. Base addition salts can be prepared by contacting the free acid form with amount of the desired base to produce the salt in the conventional manner. The free acid form may be regenerated by contacting the salt form with an acid and isolating the free acid in the conventional manner.

In one embodiment of the present invention, the pharmaceutically acceptable salt is a hydrochloride salt, such as paroxetine hydrochloride.

The term "solvate" is to be understood as meaning any form of the compound according to the present invention in which the compound is attached to another molecule via a non-covalent bond (usually a polar solvent) and includes in particular, hydrates and alcoholates, for example, methanolates. Preferred solvates are hydrates.

Any compound referred to herein is intended to represent such specific compound and certain variations or forms thereof. Specifically, the compounds referred to herein may have asymmetric centers and thus exist in different enantiomeric or diastereomeric forms. Thus, any given compound referred to herein represents any one of a racemate, one or more enantiomeric forms, one or more diastereomeric forms, and mixtures thereof. Likewise, stereoisomers or geometric isomers of double bonds may also be present, whereby in some cases the molecule may exist as an (E)-isomer or as a (Z)-isomer (trans and cis isomers). If a molecule contains multiple double bonds, each double bond will have its own stereoisomerism, which may be the same as or different from the stereoisomerism of the other double bonds of the molecule. In addition, compounds referred to herein may exist as atropisomers. All stereoisomers of the compounds referred to herein, including enantiomers, diastereomers, geometric isomers and atropisomers, and mixtures thereof, are within the scope of the present invention.

Unless otherwise indicated, the compounds of the present invention also include isotopically-labeled forms, that is, compounds which differ only in the presence of one or more isotopically-enriched atoms. For example, compounds having the present structures except for the replacement of at least one hydrogen atom with deuterium or tritium, or the replacement of at least one carbon with a ¹³C-or ¹⁴C-enriched carbon, or the replacement of at least one nitrogen with a ¹⁵N-enriched nitrogen are within the scope of the present invention.

The term "xerophthalmia" refers to a variety of factors caused by dry eyes as the main symptom of tear secretion disorders, often accompanied by itching, foreign body sensation, burning sensation in both eyes, photophobia, blurred vision, vision fluctuations, and other manifestations. Common symptoms include dry eyes, easy fatigue, eye itching, foreign body sensation, pain and burning sensation, thick secretions, fear of wind and light, and sensitivity to topical stimuli; sometimes the eyes are too dry and lack basic tears, instead of stimulating reflex tear secretion, resulting in frequent lacrimation; in the more serious cases, the eyes will be red and swollen, congestion, keratinization, corneal epithelium broken and filiform substance adhesion, and the long-term damage can cause corneal and conjunctival lesions and affect vision. The xerophthalmia of the present invention includes keratoconjunctivitis sicca (KCS) and further includes any one type of xerophthalmia of reduced tear secretion type and hyper-evaporative tear type.

The xerophthalmia with reduced tear secretion is classified into xerophthalmia with Sjogren's syndrome and xerophthalmia without Sjogren's syndrome. The xerophthalmia with a Sjogren's syndrome type, includes congenital anacryadenopathy, sarcoidosis, graft versus host (GVHD) disease resulting from bone marrow transplantation; accompanied by ocular pemphigus, Stevens-Johnson syndrome, trachoma, or the like; diabetes, laser (-assisted) in Situ Keratomileusis (LASIK), or the like are causes of decreased reflex secretion.

In addition, the xerophthalmia with hyper-evaporative tear type may include a condition accompanied by a decrease in the oil layer due to meibomian gland insufficiency, blepharitis, or the like; accompanied by blink insufficiency or eyelid closure insufficiency due to eyeball protrusion, rabbit eye, or the like; with decreased tear stability due to contact lens wear; accompanied by decreased mucin secretion from embryonic cells; conditions accompanied by VDT operation, or the like.

The term "prevention" or "treatment" includes therapeutic or prophylactic treatments or measures, the goal being to prevent or slow down a targeted pathological condition or disorder. A subject is successfully "prevented" or "treated" if, following administration of a therapeutically effective amount of a CYP450 inhibitor, pharmaceutical composition, or drug of the present invention according to the methods of the present invention, the subject exhibits an observable and/or measurable reduction or disappearance of one or more signs and symptoms of a particular disease.

In the present invention, the term "animal" generally refers to vertebrates, particularly mammals, including humans. The term "non-human animal" refers to any vertebrate animal other than a human, particularly a mammal. In some embodiments of the present invention, the non-human animal of the present invention is a domesticated animal, that is, an animal that has been raised and domesticated by humans and whose reproduction can be controlled artificially, for functions such as eating, labor, fur, pets, experimentation, for example, commercial animals, pet animals, laboratory animals, or the like. Economic animals include such as domestic animals, for example, pigs, cattle, sheep, horses, donkeys, foxes, raccoon dogs, mink, camels, or the like. Pet animals include such as dogs, cats, rabbits, mice (for example, guinea pigs, hamsters, gerbils, chinchillas, squirrels, or the like), or the like. Experimental animals include such as monkeys, dogs, rabbits, cats, and mice (for example, rats or murine), or the like.

The disclosures of the various publications, patents, and published patent specifications cited herein are hereby incorporated by reference in their entirety.

The technical solutions in the embodiments of the present invention are clearly and completely described below with reference to FIGs. 1 to 9, and it is obvious that the described embodiments are only a part of the embodiments of the present invention, and not all of the embodiments. Based on the embodiments of the present application, all other embodiments obtained by those skilled in the art without an inventive step shall fall within the protection scope of the present application.

### Example 1: Acaricidal experiment

### 1. Experimental object

This study strictly followed the ethical principles of the Declaration of Helsinki, and Ethical Guidelines for Biomedical Research Involving Human Subjects. In this study, Demodex was collected from patients diagnosed with ocular Demodex infection. Those meeting the inclusion criteria would be enrolled after an informed conversation and signing an informed consent form.

### 2. Demodex detection

Demodex detection was performed according to the conventional eyelash microscopic examination method. 3 eyelashes were extracted from each eyelid, and a total of 12 eyelashes were extracted. The extracted eyelashes were immediately placed on a glass slide, and one piece of each three eyelashes was placed under an ordinary light microscope for observation. Demodex at all stages were counted and classified according to morphology (specific criteria were: Demodex brevis at a head-to-body ratio of 1:1 and Demodex folliculorum at a head-to-body ratio of 1:3 to 1:4). Only adults that were fully exposed to the field of view were taken as subjects (larvae and eggs were not considered as subjects since they were more fragile in early life).

### 3. Demodex culture in vitro

Based on the above detection of Demodex, 50 µl of different solutions were added to each slide, and the survival of the worms was observed every 1 hour. The death was judged by observing whether the worms were active (body, limbs, or the like) under a microscope. Two skilled Demodex-related experimenters observed and judged separately during the experiment. If the judgment results were different, the third skilled experimenter was asked to make an independent judgment. The culture in vitro was performed in a climate chamber at 20°C and 96% humidity. Slides were transported in a humid chamber under observation and the solution was added appropriately to ensure high humidity before vacuoles appeared on solution evaporation.

### 4. Screening of anti-mite compounds

(1) Compound solution preparation: The compound (the structure is shown in V-A, IV-B, V-C, V-D, VI-E, and IV-F) or salt thereof was taken to prepare the solution with 0.9% NaCl solution, and the final concentrations were shown in Table 1;
(2) 50 µl of each concentration of the compound solution was added dropwise to the mites, and coverslips were covered;
(3) 50 µl of the solute of the compound, that is, 0.9% NaCl solution, was added dropwise to the control group, and the coverslips were covered;
(4) The number of mites and dosing time were recorded and the mites were placed in a climate chamber for cultivation;
(5) The activity of the mites was observed under a microscope regularly until the mites died;
(6) The time of death of the mites was recorded;
(7) The anti-mite activity of the compounds was statistically summarized.

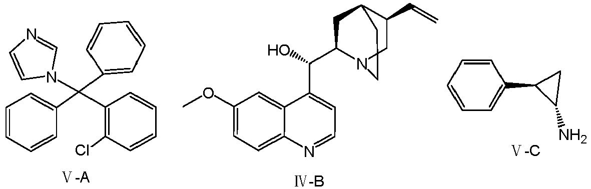

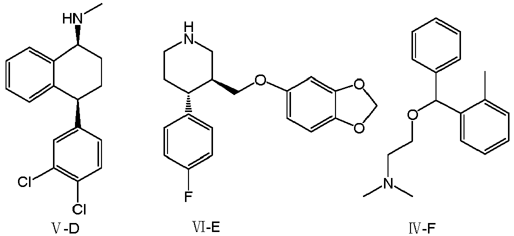

The results are shown in Table 1.

**Table 1. Results of the acaricidal experiment**

| Compound | Concentration | Average survival time of mites | Average survival time of mites in the control group |
|---|---|---|---|
| V-A | 3 mg/mL | ∼23.63 h | 61.01±14.80h |
| | 0.3 mg/mL | ∼33.01 h | 61.01±14.80h |
| Sulfate salt of IV-B | 10 mg/mL | 5h | >72h |
| | 5 mg/mL | 5.35±2.09h | 61.01±14.80h |
| | 1 mg/mL | 8.33±4.02h | 61.01±14.80h |
| Hydrochloride salt of V-C | 30 mg/mL | 2.24±1.32h | 61.01±14.80h |
| | 10 mg/mL | 2.89±1.08h | 61.01±14.80h |
| | 3 mg/mL | 5.66±0.91h | 61.01±14.80h |
| | 1 mg/mL | 7.44±6.07h | 61.01±14.80h |
| Hydrochloride salt of V-D | 10 mg/mL | 2.50±1.37h | ∼37.27±17.51 h |
| | 1 mg/mL | 9.07±6.72h | ∼37.27±17.51 h |
| VI-E | 1 mg/mL | 2.44±2.04h | 64.56±21.78h |
| Citrate salt of IV-F | 10 mg/mL | 4.35±2.05h | 45.92±23.60h |
| | 1 mg/mL | ∼7.09±1.44 h | 45.92±23.60h |

### Example 2: Clinical study on treatment of dry eye caused by mite infection

### (1) Inclusion and exclusion criteria

### 1. Case inclusion criteria

(1) Patients who meet the dry eye diagnostic criteria after inquiry of medical history;
(2) 18 to 70 years old;
(3) any gender;
(4) Patients who can cooperate with the treatment;

### 2. Case exclusion criteria

(1) Slit lamp examination for patients with iridescent cyclitis;
(2) Patients with high or low intraocular pressure
(3) Patients with ulceration wound on eyelid skin and corneal epithelial infiltration lesion on corneal surface
(4) Exclusion of systemic diseases such as Sjogren's syndrome in patients with impaired liver and kidney function;
(5) Patients under 18 years old or over 70 years old;
(6) Those whose mental state does not allow them to cooperate in the evaluation;
(7) Pregnant and lactating women.

### 3. Shedding and elimination criteria:

(1) Patients who are unable or unwilling to continue treatment due to other diseases during treatment;
(2) Patients who cannot cooperate or whose symptoms worsen during treatment and are unwilling to continue treatment;
(3) Those who violate the study protocol and use other drugs that are not used in this study;
(4) Those with incomplete final information to determine efficacy.

### (II) Endpoint indicators

Before treatment and weekly after treatment, the patient had ocular surface discomfort score, general ophthalmologic examination (visual acuity, intraocular pressure, and slit lamp examination), three tests for dry eye (conjunctival hyperemia score, BUT test, and Schirmer I test), ocular surface disease index (OSDI) score, dry eye analyzer, and ocular Demodex examination.

### (III) Data statistics

### 1. Sample size estimation

Considering that dry eye was a common ocular surface disease in the clinic, the positive rate of ocular Demodex reached 23.8% to 90.0%. Using the formula of sample size calculation for the validity test of quantitative data, it was concluded that there were 30 cases in the experimental group and 30 cases in the control group.

### 2. Statistics and analysis of study data

SPSS20.0 software and Excel were used for statistical processing. Paired sample t-test was used for normal distribution data of count data, the χ2 test was used for measurement data, and the non-parametric test was used for non-normal distribution data. The results were expressed as mean ± standard deviation (x + s), with P <0.05 as the difference.

The above is only a preferred example of the present invention and is not intended to limit the present invention, and any modifications, equivalent substitutions, or the like made within the spirit and principles of the present invention shall be included in the scope of protection of the present invention.

The foregoing examples and methods described in the present invention may vary based on the ability, experience, and preferences of the skilled in the art.

The mere listing of the steps of the method in a certain order in the present invention does not constitute any limitation on the order of the steps of the method.

## Claims

1. Use of a compound, or a salt, stereoisomer and solvate thereof, in the preparation of products for inhibiting and/or killing mites, wherein the compound is shown in the following formula: 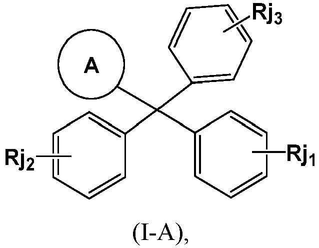
wherein Rj₁, Rj₂, and Rj₃ are one or more arbitrary and independent substituents on the corresponding phenyl group; Rj₁, Rj₂, and Rj₃ each is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; and
ring A is a heterocyclic group.

2. The use according to claim 1, **characterized in that**, the ring A is a five-membered or six-membered heteroaryl;
preferably, the ring A is 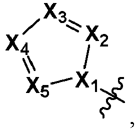 wherein, X₁, X₂, X₃, X₄, and X₅ are independently selected from CRj₄, or N;
Rj₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; or,
the ring A is 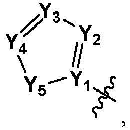 wherein, Y₁, Y₂, Y₃, and Y₄ are independently selected from CRj₅ or N; Y₅ is selected from O, S, NRjs, or CRj₅Rj₆; and
Rj₅ and Rj₆ are independently of each other selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

3. The use according to claim 2, **characterized in that**, the ring A is selected from

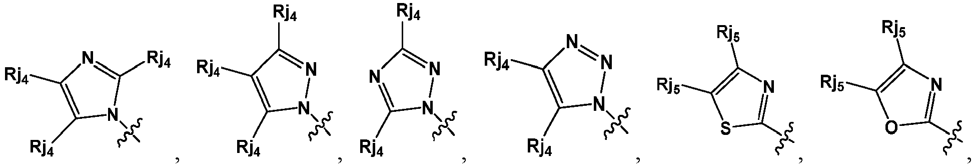

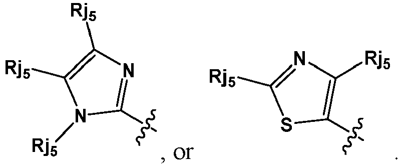

4. The use according to claim 1, **characterized in that**, Rj₁, Rj₂, and Rj₃ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; and
preferably, Rj₁ is selected from H, F, Cl, Br, or I.

5. The use according to claim 2, **characterized in that**, the compound has the following structure:

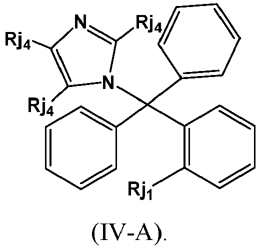

6. The use according to claim 1, **characterized in that**, the compound has the following structure:

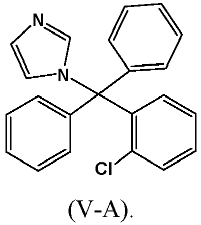

7. Use of a compound, or a salt, an ester, a stereoisomer and a solvate thereof, in the preparation of products for inhibiting and/or killing mites, wherein a structure of the compound is shown in formula I-B: 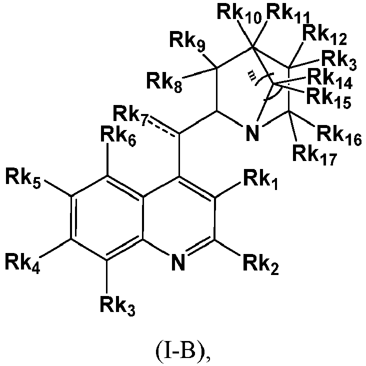 wherein,
Rk₁, Rk₂, Rk₃, Rk₄, Rk₅, and Rk₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; or, Rk₁ and Rk₂ form an aryl or heterocyclic group together with carbon atoms to which they are attached; two of Rk₃, Rk₄, Rk₅, and Rk₆ form an aryl or heterocyclic group together with carbon atoms to which they are attached;
----- represents a single bond or a double bond;
Rk₇ is selected from H, O, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rk₈, Rk₉, Rk₁₀, Rk₁₁, Rk₁₂, Rk₁₃, Rk₁₄, Rk₁₅, Rk₁₆, and Rk₁₇ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; and
m is selected from an integer from 1 to 5.

8. The use according to claim 7, **characterized in that**, Rk₁, Rk₂, Rk₃, Rk₄, and Rk₆ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

9. The use according to claim 7, **characterized in that**, Rk₅ and Rk₇ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rk₅ is OH or O(C₁₋₈ alkyl); and
preferably, Rk₇ is OH or O(C₁₋₈ alkyl).

10. The use according to claim 7, **characterized in that**, Rk₈, Rk₉, Rk₁₀, Rk₁₁, Rk₁₂, Rk₁₄, Rk₁₅, Rk₁₆, and Rk₁₇ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

11. The use according to claim 7, **characterized in that**, Rk₁₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, or C₂₋₈ alkenyl;
preferably, Rk₁₃ is selected from C₂₋₈ alkenyl; and
more preferably, Rk₁₃ is ethenyl.

12. The use according to claim 9 or 10, **characterized in that**, the compound has the following structure:

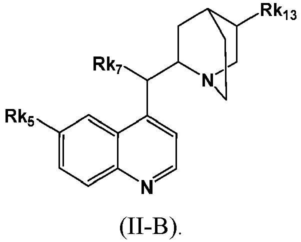

13. The use according to claim 7, **characterized in that**, the compound has the following structure:

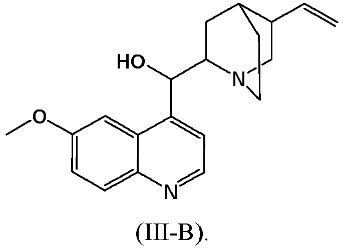

14. The use according to claim 7, **characterized in that**, the compound has the following structure:

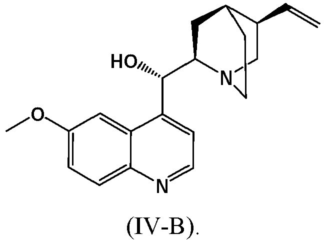

15. The use according to claim 7, **characterized in that**, the salt of the compound is a sulfate salt thereof.

16. Use of a compound, or a salt, an ester, a stereoisomer and a solvate thereof, in the preparation of products for inhibiting and/or killing mites, wherein a structure of the compound is shown in formula I-C: 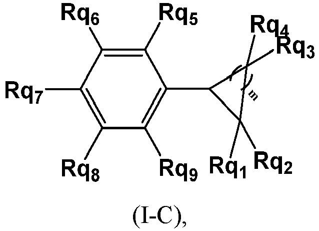 wherein,
Rq₁, Rq₂, Rq₃, and Rq₄ are each independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rq₅, Rq₆, Rq₇, Rq₈, and Rq₉ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), NHC(=O)(C₆₋₁₈ aryl), NHC(=O)(C1-10 aralkyl), NHC(=O)(C1-10 arylalkoxy), N(C₁₋₈ alkyl)C(=O)(C₁₋₈ alkyl), NHC(=O)(C₁₋₈ alkyl)NHC(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, two of Rq₁, Rq₂, Rq₃, Rq₄, Rq₅, Rq₆, Rq₇, Rq₈, and Rq₉ form an aryl, cycloalkyl, or heterocyclic group together with carbon atoms to which they are attached; and
m is selected from an integer from 1 to 5.

17. The use according to claim 16, **characterized in that**, Rq₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rq₁ is selected from NH₂, NH(C₁₋₈ alkyl), or N(C₁₋₈ alkyl)₂; and
more preferably, Rq₁ is NH₂.

18. The use according to claim 16, **characterized in that**, Rq₇ is selected from H, F, Cl, Br, I, C₁₋₈ alkyl, O(C₁₋₈ alkyl), NH(C₁₋₈ alkyl), N(C₁₋₈ alkyl)₂, NHC(=O)(C₁₋₈ alkyl), NHC(=O)(C₆₋₁₈ aryl), NHC(=O)(C1-10 aralkyl), NHC(=O)(C1-10 arylalkoxy), OH, NH₂, SH, CO₂H, CN, CF₃, CHF₂, CH₂F, or NO₂.

19. The use according to claim 16, **characterized in that**, Rq₂, each Rq₃, each Rq₄, Rq₅, Rq₆, Rq₈, and Rq₉ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

20. The use according to claim 16, **characterized in that**, the compound has the following structure:

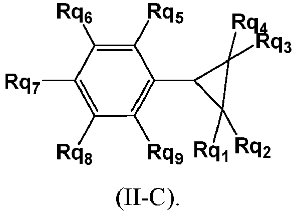

21. The use according to claim 17, **characterized in that**, the compound has the following structure:

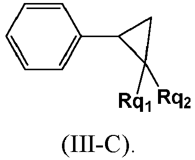

22. The use according to claim 16, **characterized in that**, the compound has the following structure:

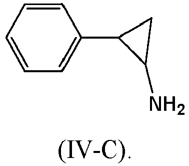

23. The use according to claim 16, **characterized in that**, the compound has the following structure:

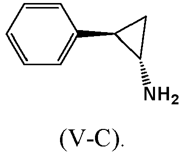

24. The use according to claim 16, **characterized in that**, the salt of the compound is a hydrochloride salt thereof.

25. Use of a compound, or a salt, an ester, a stereoisomer and a solvate thereof, in the preparation of products for inhibiting and/or killing mites, wherein a structure of the compound is shown in formula I-D: 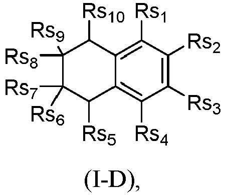 wherein, Rs₁, Rs₂, Rs₃, Rs₄, Rs₅, Rs₆, Rs₇, Rs₈, Rs₉, and Rs₁₀ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂.

26. The use according to claim 25, **characterized in that**, the compound has the following structure: 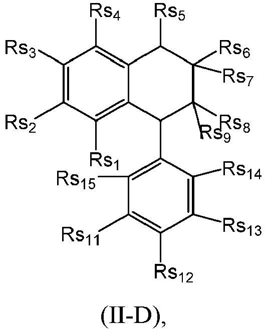 wherein, Rs₁₁, Rs₁₂, Rs₁₃, Rs₁₄, and Rs₁₅ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂.

27. The use according to claim 25, **characterized in that**, Rs₁, Rs₂, Rs₃, Rs₄, Rs₆, Rs₇, Rs₈, Rs₉, Rs₁₃, Rs₁₄, and Rs₁₅ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl.

28. The use according to claim 25, **characterized in that**, Rss is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
preferably, Rss is selected from NH(C₁₋₈ alkyl); and
more preferably, Rss is NHCH₃.

29. The use according to claim 25, **characterized in that**, Rs₁₁ and Rs₁₂ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
preferably, Rs₁₁ is H, F, Cl, Br, and I;
preferably, Rs₁₂ is H, F, Cl, Br, and I.

30. The use according to claim 28 or 29, **characterized in that**, the compound has the following structure:

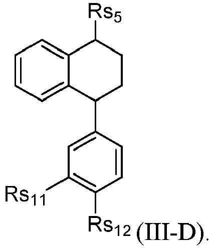

31. The use according to claim 25, **characterized in that**, the compound has the following structure:

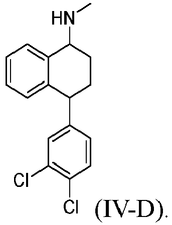

32. The use according to claim 25, **characterized in that**, the compound has the following structure:

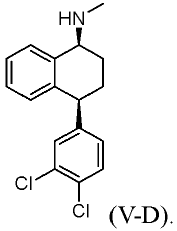

33. The use according to claim 25, **characterized in that**, the salt of the compound is a hydrochloride salt thereof.

34. Use of a compound, or a salt, an ester, a stereoisomer and a solvate thereof, in the preparation of products for inhibiting and/or killing mites, wherein a structure of the compound is shown in formula I-E: 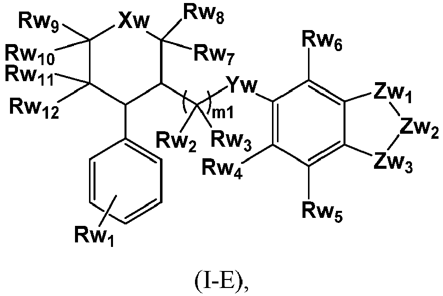 wherein,
Rw₁ is one or more arbitrary and independent substituents on the phenyl, each Rw₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rw₂, Rw₃, Rw₄, Rw₅, Rw₆, Rw₇, Rw₈, Rw₉, Rw₁₀, Rw₁₁, and Rw₁₂ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, Rw₃ and Rw₄, or Rw₄ and Rw₅ form an aryl, cycloalkyl or heterocyclic group together with carbon atoms to which they are attached;
Xw, Yw, Zw₁, Zw₂, and Zw₃ are independently selected from CRw₁₃Rw₁₄, O, S, or NRw₁₃;
m1 is selected from an integer from 1 to 5; and
Rw₁₃ and Rw₁₄ are independently of each other selected from H, halogen, C₁₋₈ alkyl, C₃₋₁₁ cycloalkyl, aryl, or heteroaryl.

35. The use according to claim 34, **characterized in that**, Rw₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rw₁ is selected from F, Cl, Br, or I; and
more preferably, Rw₁ is F.

36. The use according to claim 34, **characterized in that**, Rw₂, Rw₃, Rw₄, Rw₅, Rw₆, Rw₇, Rw₈, Rw₉, Rw₁₀, R_{w11}, and Rw₁₂ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

37. The use according to claim 34, **characterized in that**, Xw is NRw₁₃; Rw₁₃ is selected from H, C₁₋₈ alkyl, or C₃₋₁₁ cycloalkyl; and
preferably, Xw is NH.

38. The use according to claim 34, **characterized in that**, Yw is O.

39. The use according to claim 34, **characterized in that**, Zw₁ and/or Zw₃ is O.

40. The use according to claim 34, **characterized in that**, Zw₂ is CRw₁₃Rw₁₄; wherein, Rw₁₃ and Rw₁₄ are independently selected from H, halogen, C₁₋₈ alkyl, C₃₋₁₁ cycloalkyl, aryl, or heteroaryl; and
preferably, Zw₂ is CH₂.

41. The use according to any one of claims 34 to 37, **characterized in that**, the compound has the following structure:

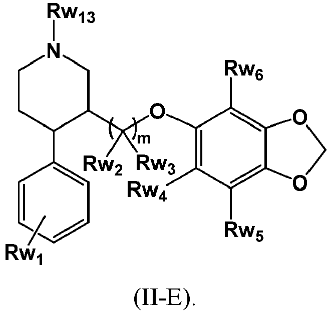

42. The use according to any one of claims 34 to 37, **characterized in that**, the compound has the following structure:

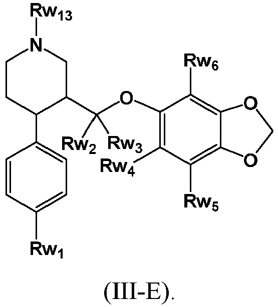

43. The use according to any one of claims 34 to 37, **characterized in that**, the compound has the following structure:

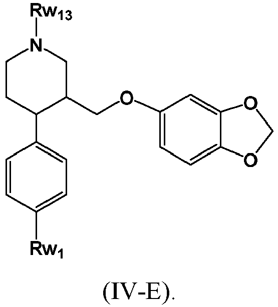

44. The use according to claim 34, **characterized in that**, the compound has the following structure:

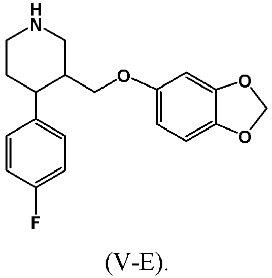

45. The use according to claim 34, **characterized in that**, the compound has the following structure:

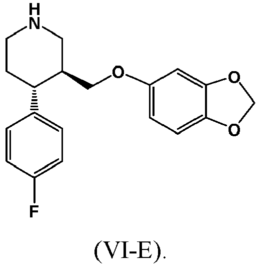

46. Use of a compound, or a salt, an ester, a stereoisomer and a solvate thereof, in the preparation of products for inhibiting and/or killing mites, wherein a structure of the compound is shown in formula I-F: 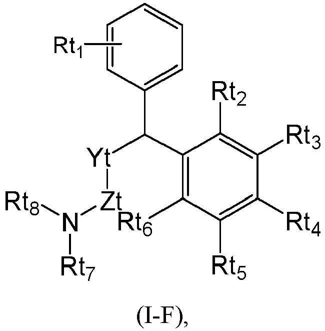 wherein,
Rt₁ is one or more arbitrary and independent substituents on the phenyl, each Rt₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₂, Rt₃, Rt₄, Rt₅, and Rt₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₇ and Rt₈ are independently selected from H, C₁₋₈ alkyl, or C₃₋₆ cycloalkyl;
Yt is selected from CRt₉Rt₁₀, O, S, or NRt₉;
Zt is C₁₋₈ alkylene; and
Rt₉ and Rt₁₀ are independently of each other selected from H, halogen, C₁₋₈ alkyl, C₃₋₁₁ cycloalkyl, aryl, or heteroaryl.

47. The use according to claim 46, **characterized in that**, each Rt₁, Rt₂, Rt₃, Rt₄, Rt₅, and Rt₆ is independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; preferably, Rt₁, Rt₃, Rt₄, Rt₅, and Rt₆ are all H; preferably, Rt₂ is methyl.

48. The use according to claim 46, **characterized in that**, Rt₇ and Rt₈ are independently selected from H or C₁₋₃ alkyl; preferably, Rt₇ and Rt₈ are C₁₋₃ alkyl; more preferably, Rt₇ and Rt₈ are methyl.

49. The use according to claim 46, **characterized in that**, Yt is selected from CH₂, O, S, or NH; preferably, Yt is O.

50. The use according to claim 46, **characterized in that**, Zt is C₁₋₆ alkylene, preferably C₁₋₃ alkylene, and more preferably -CH₂CH₂-.

51. The use according to any one of claims 46 to 50, **characterized in that**, the compound has the following structure:

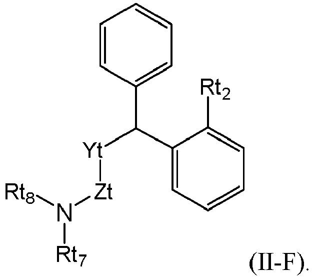

52. The use according to any one of claims 46 to 50, **characterized in that**, the compound has the following structure:

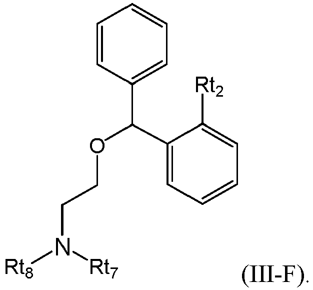

53. The use according to claim 46, **characterized in that**, the compound has the following structure:

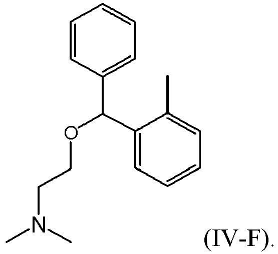

54. The use according to claim 46, **characterized in that**, the salt of the compound is a citrate salt thereof.

55. The use according to any one of claims 1 to 54, **characterized in that**, the products are pharmaceutical compositions, cosmetics, or acaricides.

56. The use according to claim 55, **characterized in that**, the pharmaceutical compositions are pharmaceutical compositions for the prevention and/or treatment of diseases caused by mite infection;
the diseases are selected from eye diseases, skin diseases, or allergic diseases;
preferably, the eye diseases are selected from blepharitis, palpebral limbic keratoconjunctivitis, meibomian gland dysfunction, eyelash loss, abnormal eyelash arrangement, conjunctivitis, or palpebral conjunctivitis, pterygium, keratitis, eyelid basal cell carcinoma, dry eye, or chalazion;
preferably, the skin diseases are selected from seborrheic dermatitis, acne, rosacea, pityriasis folliculitis, perioral dermatitis, Demodex diseases, scabies, or basal cell carcinoma; and
preferably, the allergic diseases are selected from allergic asthma, allergic rhinitis, allergic dermatitis, or allergic conjunctivitis.

57. The use according to claim 55, **characterized in that**, the pharmaceutical compositions are topical preparations, and preferably ophthalmic preparations or topical dermatological preparations;
preferably, the ophthalmic preparations are selected from eye drops, eye ointments, ophthalmic gels, ophthalmic emulsions, ophthalmic suspensions, ophthalmic films, ophthalmic solutions, or intraocular injections; and
preferably, the topical dermatological preparations are selected from aerosols, powders, lotions, tinctures, liniments, films, ointments, gels, pastes, or emulsions.

58. The use according to claim 55, **characterized in that**, the pharmaceutical compositions are for human and/or non-human animals; and
preferably, the non-human animals are pet animals or livestock animals.

59. Use of a compound, or a salt, stereoisomer, and solvate thereof in the preparation of drugs for preventing and/or treating xerophthalmia, wherein the compound is shown in the following formula: 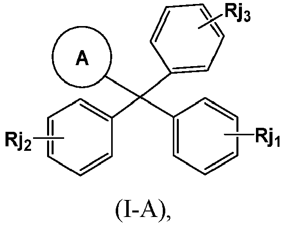
wherein Rj₁, Rj₂, and Rj₃ are one or more arbitrary and independent substituents on the corresponding phenyl group; Rj₁, Rj₂, and Rj₃ each is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; and
ring A is a heterocyclic group.

60. The use according to claim 59, **characterized in that**, the ring A is a five-membered or six-membered heteroaryl;
preferably, the ring A is 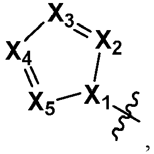 wherein, X₁, X₂, X₃, X₄, and X₅ are independently selected from CRj₄, or N;
Rs₄ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; or,
the ring A is 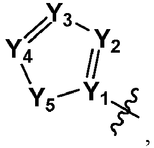 wherein, Y₁, Y₂, Y₃, and Y₄ are independently selected from CRj₅ or N; Y₅ is selected from O, S, NRj₅, or CRj₅Rj₆; and
Rj₅ and Rj₆ are independently of each other selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

61. The use according to claim 60, **characterized in that**, the ring A is selected from

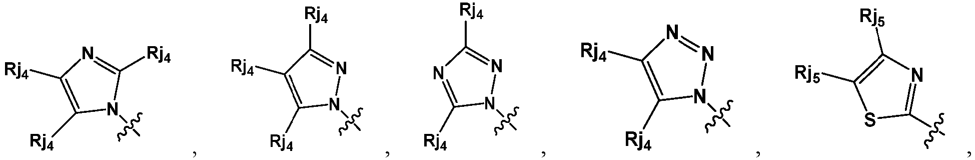

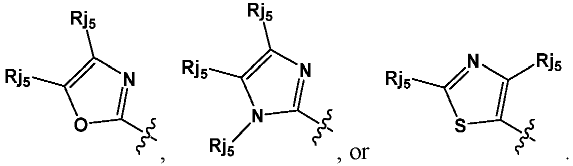

62. The use according to claim 59, **characterized in that**, Rj₁, Rj₂, and Rj₃ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; and
preferably, Rj₁ is selected from H, F, Cl, Br, or I.

63. The use according to claim 60, **characterized in that**, the compound has the following structure:

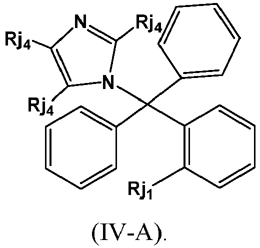

64. The use according to claim 59, **characterized in that**, the compound has the following structure:

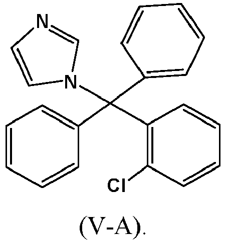

65. Use of a compound, or a salt, an ester, a stereoisomer and a solvate thereof, in the preparation of drugs for preventing and/or treating xerophthalmia, wherein a structure of the compound is shown in formula I-B: 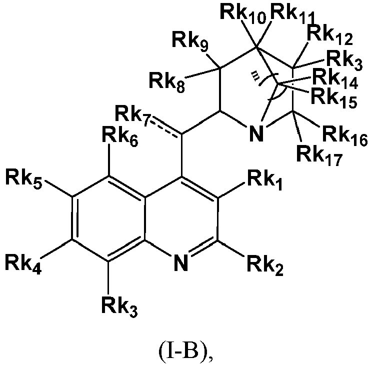 wherein,
Rk₁, Rk₂, Rk₃, Rk₄, Rk₅, and Rk₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; or, Rk₁ and Rk₂ form an aryl or heterocyclic group together with carbon atoms to which they are attached; two of Rk₃, Rk₄, Rk₅, and Rk₆ form an aryl or heterocyclic group together with carbon atoms to which they are attached;
----- represents a single bond or a double bond;
Rk₇ is selected from H, O, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋s alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rk₈, Rk₉, Rk₁₀, Rk₁₁, Rk₁₂, Rk₁₃, Rk₁₄, Rk₁₅, Rk₁₆, and Rk₁₇ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂; and
m is selected from an integer from 1 to 5.

66. The use according to claim 65, **characterized in that**, Rk₁, Rk₂, Rk₃, Rk₄, and Rk₆ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

67. The use according to claim 65, **characterized in that**, Rk₅ and Rk₇ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rk₅ is OH or O(C₁₋₈ alkyl); and
preferably, Rk₇ is OH or O(C₁₋₈ alkyl).

68. The use according to claim 65, **characterized in that**, Rk₈, Rk₉, Rk₁₀, Rk₁₁, Rk₁₂, Rk₁₄, Rk₁₅, Rk₁₆, and Rk₁₇ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

69. The use according to claim 65, **characterized in that**, Rk₁₃ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, or C₂₋₈ alkenyl;
preferably, Rk₁₃ is selected from C₂₋₈ alkenyl; and
more preferably, Rk₁₃ is ethenyl.

70. The use according to claim 67 or 69, **characterized in that**, the compound has the following structure:

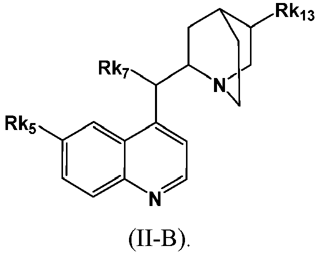

71. The use according to claim 65, **characterized in that**, the compound has the following structure:

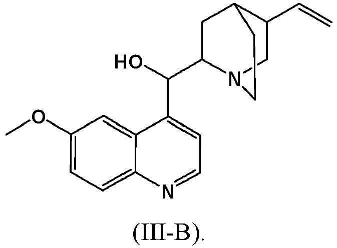

72. The use according to claim 65, **characterized in that**, the compound has the following structure:

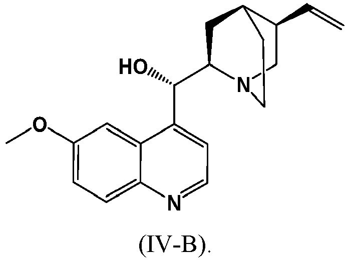

73. The use according to claim 65, **characterized in that**, the salt of the compound is a sulfate salt thereof.

74. Use of a compound, or a salt, an ester, a stereoisomer and a solvate thereof, in the preparation of drugs for preventing and/or treating xerophthalmia, wherein a structure of the compound is shown in formula I-C: 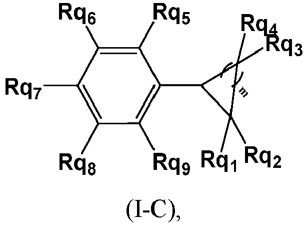 wherein,
Rq₁, Rq₂, Rq₃, and Rq₄ are each independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rq₅, Rq₆, Rq₇, Rq₈, and Rq₉ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), NHC(=O)(C₆₋₁₈ aryl), NHC(=O)(C1-10 aralkyl), NHC(=O)(C1-10 arylalkoxy), N(C₁₋₈ alkyl)C(=O)(C₁₋₈ alkyl), NHC(=O)(C₁₋₈ alkyl)NHC(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, two of Rq₁, Rq₂, Rq₃, Rq₄, Rq₅, Rq₆, Rq₇, Rq₈, and Rq₉ form an aryl, cycloalkyl, or heterocyclic group together with carbon atoms to which they are attached; and
m is selected from an integer from 1 to 5.

75. The use according to claim 74, **characterized in that**, Rq₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rq₁ is selected from NH₂, NH(C₁₋₈ alkyl), or N(C₁₋₈ alkyl)₂; and
more preferably, Rq₁ is NH₂.

76. The use according to claim 74, **characterized in that**, Rq₇ is selected from H, F, Cl, Br, I, C₁₋₈ alkyl, O(C₁₋₈ alkyl), NH(C₁₋₈ alkyl), N(C₁₋₈ alkyl)₂, NHC(=O)(C₁₋₈ alkyl), NHC(=O)(C₆₋₁₈ aryl), NHC(=O)(C1-10 aralkyl), NHC(=O)(C1-10 arylalkoxy), OH, NH₂, SH, CO₂H, CN, CF₃, CHF₂, CH₂F, or NO₂.

77. The use according to claim 74, **characterized in that**, Rq₂, each Rq₃, each Rq₄, Rq₅, Rq₆, Rq₈, and Rq₉ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

78. The use according to claim 74, **characterized in that**, the compound has the following structure:

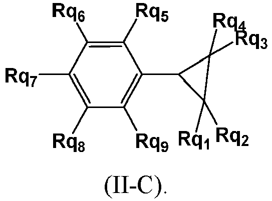

79. The use according to claim 75, **characterized in that**, the compound has the following structure:

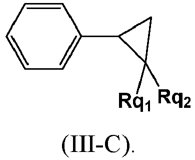

80. The use according to claim 74, **characterized in that**, the compound has the following structure:

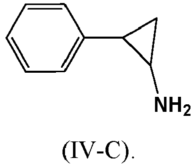

81. The use according to claim 74, **characterized in that**, the compound has the following structure:

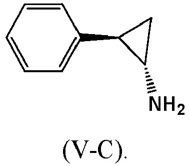

82. The use according to claim 74, **characterized in that**, the salt of the compound is a hydrochloride salt thereof.

83. Use of a compound, or a salt, an ester, a stereoisomer and a solvate thereof, in the preparation of drugs for preventing and/or treating xerophthalmia, wherein a structure of the compound is shown in formula I-D: 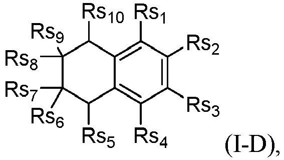 wherein, Rs₁, Rs₂, Rs₃, Rs₄, Rs₅, Rs₆, Rs₇, Rs₈, Rs₉, and Rs₁₀ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂.

84. The use according to claim 83, **characterized in that**, the compound has the following structure: 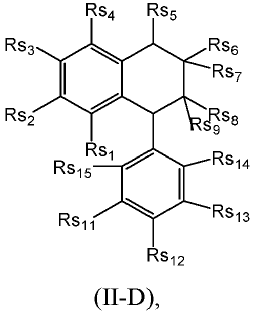 wherein, Rs₁₁, Rs₁₂, Rs₁₃, Rs₁₄, and Rs₁₅ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂.

85. The use according to claim 83, **characterized in that**, Rs₁, Rs₂, Rs₃, Rs₄, Rs₆, Rs₇, Rs₈, Rs₉, Rs₁₃, Rs₁₄, and Rs₁₅ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl.

86. The use according to claim 83, **characterized in that**, Rss is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
preferably, Rss is selected from NH(C₁₋₈ alkyl); and
more preferably, Rss is NHCH₃.

87. The use according to claim 83, **characterized in that**, Rs₁₁ and Rs₁₂ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, NO₂, C₂₋₈ alkenyl, or C₂₋₈ alkynyl;
preferably, Rs₁₁ is H, F, Cl, Br, and I;
preferably, Rs₁₂ is H, F, Cl, Br, and I.

88. The use according to claim 86 or 87, **characterized in that**, the compound has the following structure:

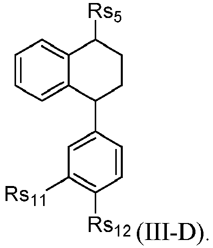

89. The use according to claim 83, **characterized in that**, the compound has the following structure:

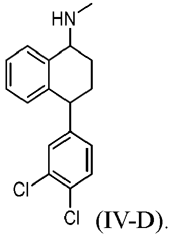

90. The use according to claim 83, **characterized in that**, the compound has the following structure:

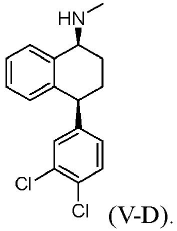

91. The use according to claim 83, **characterized in that**, the salt of the compound is a hydrochloride salt thereof.

92. Use of a compound, or a salt, stereoisomer, and solvate thereof in the preparation of drugs for preventing and/or treating xerophthalmia, wherein a structure of the compound is shown in formula I-E: 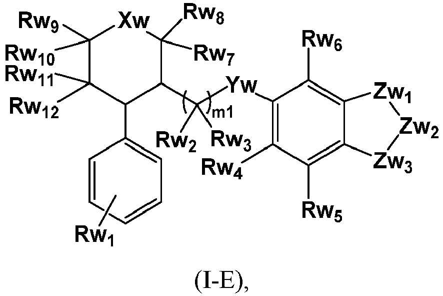 wherein,
Rw₁ is one or more arbitrary and independent substituents on the phenyl, each Rw₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rw₂, Rw₃, Rw₄, Rw₅, Rw₆, Rw₇, Rw₈, Rw₉, Rw₁₀, Rw₁₁, and Rw₁₂ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
or, Rw₃ and Rw₄, or Rw₄ and Rw₅ form an aryl, cycloalkyl or heterocyclic group together with carbon atoms to which they are attached;
Xw, Yw, Zw₁, Zw₂, and Zw₃ are independently selected from CRw₁₃Rw₁₄, O, S, or NRw₁₃;
m1 is selected from an integer from 1 to 5; and
Rw₁₃ and Rw₁₄ are independently of each other selected from H, halogen, C₁₋₈ alkyl, C₃₋₁₁ cycloalkyl, aryl, or heteroaryl.

93. The use according to claim 92, **characterized in that**, Rw₁ is selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂;
preferably, Rw₁ is selected from F, Cl, Br, or I; and
more preferably, Rw₁ is F.

94. The use according to claim 92, **characterized in that**, Rw₂, Rw₃, Rw₄, Rw₅, Rw₆, Rw₇, Rws, Rw₉, Rw₁₀, Rw₁₁, and Rw₁₂ are independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂.

95. The use according to claim 92, **characterized in that**, Xw is NRw₁₃; Rw₁₃ is selected from H, C₁₋₈ alkyl, or C₃₋₁₁ cycloalkyl; and
preferably, Xw is NH.

96. The use according to claim 92, **characterized in that**, Yw is O.

97. The use according to claim 92, **characterized in that**, Zw₁ and/or Zw₃ is O.

98. The use according to claim 92, **characterized in that**, Zw₂ is CRw₁₃Rw₁₄; wherein, Rw₁₃ and Rw₁₄ are independently selected from H, halogen, C₁₋₈ alkyl, C₃₋₁₁ cycloalkyl, aryl, or heteroaryl; and
preferably, Zw₂ is CH₂.

99. The use according to any one of claims 92 to 95, **characterized in that**, the compound has the following structure:

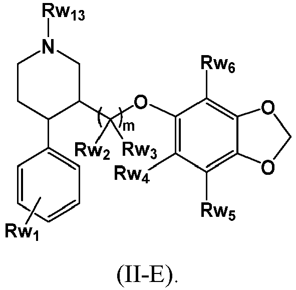

100. The use according to any one of claims 92 to 95, **characterized in that**, the compound has the following structure:

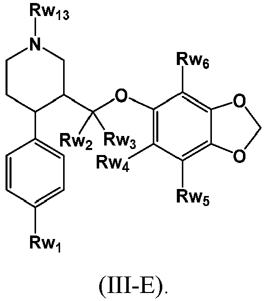

101. The use according to any one of claims 92 to 95, **characterized in that**, the compound has the following structure:

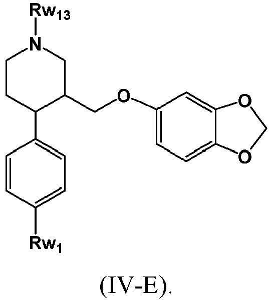

102. The use according to claim 92, **characterized in that**, the compound has the following structure:

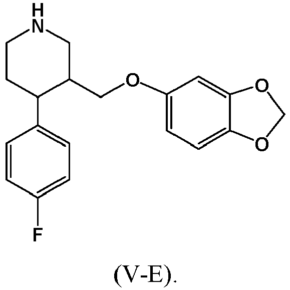

103. The use according to claim 92, **characterized in that**, the compound has the following structure:

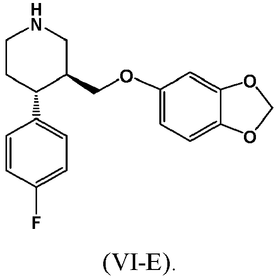

104. Use of a compound, or a salt, stereoisomer, and solvate thereof in the preparation of drugs for preventing and/or treating xerophthalmia, wherein a structure of the compound is shown in formula I-F: 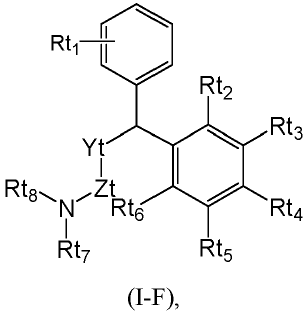 wherein,
Rt₁ is one or more arbitrary and independent substituents on the phenyl, each Rt₁ is independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₂, Rt₃, Rt₄, Rts, and Rt₆ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, NHC(=O)(C₁₋₈ alkyl), N(C₁₋₈ alkyl)C(=O)(C₁₋₈ alkyl), N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), or NHSO₂N(C₁₋₈ alkyl)₂;
Rt₇ and Rt₈ are independently selected from H, C₁₋₈ alkyl, or C₃₋₆ cycloalkyl;
Yt is selected from CRt₉Rt₁₀, O, S, or NRt₉;
Zt is C₁₋₈ alkylene; and
Rt₉ and Rt₁₀ are independently of each other selected from H, halogen, C₁₋₈ alkyl, C₃₋₁₁ cycloalkyl, aryl, or heteroaryl.

105. The use according to claim 104, **characterized in that**, each Rt₁, Rt₂, Rt₃, Rt₄, Rt₅, and Rt₆ is independently selected from H, F, Cl, Br, I, C₁₋₈ alkyl, O(C₁₋₈ alkyl), NH(C₁₋₈ alkyl), N(C₁₋₈ alkyl)₂, OH, NH₂, SH, CO₂H, CN, CF₃, CHF₂, CH₂F, or NO₂; preferably, Rt₁, Rt₃, Rt₄, Rt₅, and Rt₆ are all H; preferably, Rt₂ is methyl.

106. The use according to claim 104, **characterized in that**, Rt₇ and Rt₈ are independently selected from H or C₁₋₃ alkyl; preferably, Rt₇ and Rt₈ are alkyl; more preferably, Rt₇ and Rt₈ are methyl.

107. The use according to claim 104, **characterized in that**, Yt is selected from CH₂, O, S, or NH; preferably, Yt is O.

108. The use according to claim 104, **characterized in that**, Zt is C₁₋₆ alkylene, preferably C₁₋₃ alkylene, and more preferably -CH₂CH₂-.

109. The use according to any one of claims 104 to 108, **characterized in that**, the compound has the following structure:

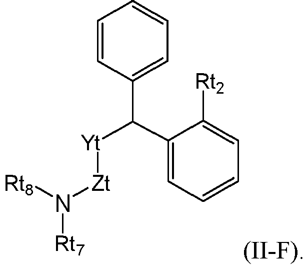

110. The use according to any one of claims 104 to 109, **characterized in that**, the compound has the following structure:

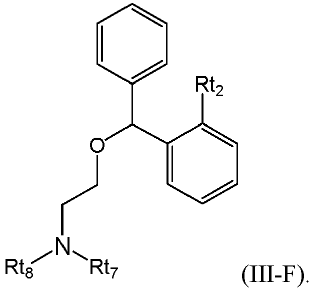

111. The use according to claim 104, **characterized in that**, the compound has the following structure:

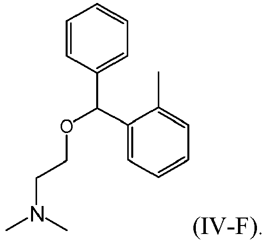

112. The use according to claim 104, **characterized in that**, the salt of the compound is a citrate salt thereof.

113. The use according to any one of claims 59 to 112, **characterized in that**, the drugs are ophthalmic preparations;
preferably, the ophthalmic preparations are selected from eye drops, eye ointments, ophthalmic gels, ophthalmic emulsions, ophthalmic suspensions, ophthalmic films, ophthalmic solutions, or intraocular injections; and
preferably, the drugs are used for human and/or non-human animals; and
preferably, the non-human animals are pet animals or livestock animals.
